(19) Europäisches Patentamt
European Patent Office
Office européen des brevets

(11) Veröffentlichungsnummer : **0 339 521 B1**

(12) **EUROPÄISCHE PATENTSCHRIFT**

(45) Veröffentlichungstag der Patentschrift :
**18.03.92 Patentblatt 92/12**

(51) Int. Cl.$^5$ : **C25B 3/02,** C07C 49/83, C07C 49/757, C07C 47/57, C07C 43/303, C07C 43/315, C07C 49/755

(21) Anmeldenummer : **89107286.0**

(22) Anmeldetag : **22.04.89**

(54) **Verfahren zur Herstellung von Tetralinderivaten und neue Tetralinderivate.**

(30) Priorität : **27.04.88 DE 3814180**

(43) Veröffentlichungstag der Anmeldung :
**02.11.89 Patentblatt 89/44**

(45) Bekanntmachung des Hinweises auf die Patenterteilung :
**18.03.92 Patentblatt 92/12**

(84) Benannte Vertragsstaaten :
**AT BE CH DE FR GB IT LI NL**

(56) Entgegenhaltungen :
**EP-A- 0 098 546**
**PATENT ABSTRACTS OF JAPAN, vol. 10, no. 144 (C-349)[2201], 27th May 1986; & JP-A-61 3892 (OOSAKA YUUKI KAGAKU KOGYO K.K.) 09-01-1986**

(73) Patentinhaber : **BASF Aktiengesellschaft**
**Carl-Bosch-Strasse 38**
**W-6700 Ludwigshafen (DE)**

(72) Erfinder : **Degner, Dieter, Dr.**
**Kurpfalzstrasse 8**
**W-6701 Dannstadt-Schauernheim (DE)**
Erfinder : **Potzolli, Bernd, Dr.**
**Sonnenwendstrasse 94**
**W-6702 Bad Duerkheim (DE)**
Erfinder : **Janitschke, Lothar, Dr.**
**Wormser Gasse 9A**
**W-6711 Kleinniedesheim (DE)**

**EP 0 339 521 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Diese Erfindung betrifft ein neues Verfahren zur Herstellung von Tetralinderivaten durch Elektrooxidation sowie neue Tetralinderivate.

Es wurde gefunden daß man Tetralinderivate der Formel

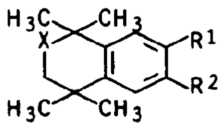

I,

in der
X eine $-CH_2-$, -CH(OH)- oder -CO-Gruppe,
$R^1$ eine der Gruppen $-COCH_3$, $-CH(OR^3)_2$ oder -CHO,
$R^2$ ein Wasserstoffatom oder die Methylgruppe und
$R^3$ einen Alkylrest mit 1 bis 4 C-Atomen
bedeuten, vorteilhaft dadurch herstellen kann, daß man Verbindungen der Formel

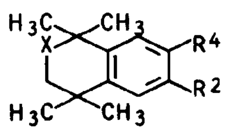

II,

in der $R^4$ eine Methyl- oder Ethylgruppe bedeutet und X und $R^2$ die oben genannte Bedeutung haben, elektrochemisch oxidiert.

Tetralinderivate ähnlicher Art werden z.B. in der DE-OS 34 34 942 beschrieben. Sie wurden bisher bevorzugt durch zweimalige Friedel-Crafts-Reaktion oder durch Friedel-Crafts-Reaktion und Grignard-Reaktion hergestellt, wobei sie in schlechter Ausbeute und mit geringer Selektivität erhalten wurden.

Die Erfindung betrifft außerdem die neuen Tetralinderivate der Formeln

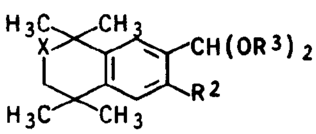

III

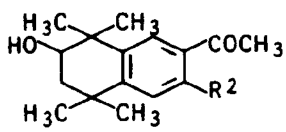

IV

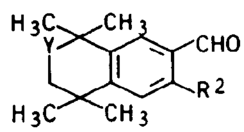

V

in denen
X eine $-CH_2-$, -CH(OH)- oder -CO-Gruppe,
$R^2$ ein Wasserstoffatom oder die Methylgruppe,
$R^3$ eine Alkylgruppe mit 1 bis 4 C-Atomen und
Y eine -CH(OH)- oder -CO-Gruppe
bedeuten.

In diesen neuen Tetralinderivaten steht der Alkylrest $R^3$ vorzugsweise für eine Methyl- oder Ethylgruppe. Die neuen Tetralinderivate sind Riechstoffe mit moschusähnlichen Eigenschaften. Weiterhin sind sie Zwischenprodukte, z.B. für die Herstellung von Wirkstoffen auf dem Pharmagebiet, insbesondere von Retinoiden. So lassen sich aus den neuen Tetralinderivaten auf an sich bekannte Weise, z.B. durch Wittig-Horner- oder Wittig-Reaktion, in einer oder mehreren Stufen Retinoide mit verbessertem Wirkungsspektrum, wie sie in der DE-OS 34 34 942 beschrieben sind, einfacher aufbauen.

Als neue Tetralinderivate seien z.B. die folgenden Verbindungen genannt: 1,2,3,4-Tetrahydro-6-dimethoximethyl-1,1,4,4-tetramethylnaphthalin, 6-Diethoximethyl-1,2,3,4-tetrahydro-1,1,4,4-tetramethylnaphthalin, 1,2,3,4-Tetrahydro-6-dimethoximethyl-1,1,4,4,7-pentamethylnaphthalin, 7-Formyl-1,2,3,4-tetrahydro-2-hydroxi-1,1,4,4-tetramethylnaphthalin, 1,2,3,4-Tetrahydro-2-hydroxi-7-dimethoximethyl-1,1,4,4-tetramethylnaphthalin, 7-Acetyl-1,2,3,4-tetrahydro-2-hydroxi-1,1,4,4-tetramethylnaphthalin, 1,2,3,4-Tetrahydro-7-dimethoximethyl-1,1,4,4-tetramethyl-2-oxo-naphthalin und 7-Formyl-1,2,3,4-tetrahydro-1,1,4,4-tetramethyl-2-oxo-naphthalin.

Die Tetralinderivate der Formel I werden erfindungsgemäß auf besonders vorteilhafte Weise durch Elektrooxidation der Alkylderivate der Formel II erhalten. Dabei verwendet man bei der Herstellung von solchen Tetralinderivaten der Formel I, die als Rest $R^1$ eine Acetylgruppe tragen, einen Elektrolyten, der die Ausgangsverbindung der Formel II, in der $R^4$ Ethyl bedeutet, in einem wäßrigen Medium enthält. Neben der Ausgangsverbindung enthält der wäßrige Elektrolyt zweckmäßigerweise noch ein Kolösungsmittel und einen Hilfselektrolyten. Als Kolösungsmittel werden solche eingesetzt, die unter den Elektrolysebedingungen stabil sind, das sind z.B. Nitrile wie Acetonitril oder Benzonitril, Ketone wie Aceton oder Methylethylketon, Ether wie Tetrahydrofuran. Bevorzugte Kolösungsmittel sind Alkanole, wie Ethanol, Isopropanol und tert. Butanol. Besonders bevorzugt ist Methanol als Kolösungsmittel. Als Hilfselektrolyte können die üblichen Verbindungen, wie Basen, Neutralsalze und Säuren eingesetzt werden. Basen sind z.B. Alkalialkoholate wie $NaOCH_3$; Neutralsalze sind z.B. Fluoride, wie KF, Sulfonate wie $KSO_3C_6H_5$ oder $NaSO_3C_6H_5$, Tetrafluorborate wie $LiBF_4$ oder Alkylsulfate wie $(CH_3)_4NSO_4CH_3$. Als Säuren dienen z.B. Sulfonsäuren wie $CH_3SO_3H$ oder $C_6H_5$-$SO_3H$ und Schwefelsäure. Bevorzugt sind Gemische aus Neutralsalzen und Säuren. Besonders bevorzugt werden Benzolsulfonate und Benzolsulfonsäuren eingesetzt. Ein für die elektrochemische Oxidation besonders geeigneter Elektrolyt ist beispielsweise wie folgt zusammengesetzt.

5 - 25 % Tetralinderivat der Formel II
5 - 30 % Wasser
40 - 80 % Alkanol
0,1 - 5 % Säure
0,5 - 5 % Neutralsalz

Bei der Herstellung von Tetralinderivaten der Formel I, in der $R^1$ einen Rest der Formel $-CH(OR^3)_2$ bedeutet, geht man von einem Elektrolyten aus, der die Ausgangsverbindung der Formel II, in der $R^4$ Methyl bedeutet, und ein Alkanol der Formel $R^3OH$, in der R für einen Alkylrest mit 1 bis 4 C-Atomen steht, enthält. Außerdem enthält der alkoholische Elektrolyt zweckmäßigerweise noch einen Hilfselektrolyten. Besonders bevorzugt wird Methanol als Alkanol eingesetzt. Als Hilfselektrolyte können auch hier Basen, Neutralsalze und Säuren, wie sie schon oben genannt wurden, eingesetzt werden. Ein für die elektrochemische Oxidation besonders geeigneter Elektrolyt ist beispielsweise wie folgt zusammengesetzt:

3 - 25 % Tetralinderivat der Formel II
30 - 90 % $R^3OH$
0,5 - 5 % Säure oder Neutralsalz sowie Gemische daraus

Die erfindungsgemäße elektrochemische Oxidation kann in den technisch üblichen Elektrolysezellen durchgeführt werden. Bevorzugt werden ungeteilte Durchflußzellen eingesetzt. Als Anodenmaterialien kommen Edelmetalle, wie Pt oder Metalloxide wie $PbO_2$ oder $RuO_2$ in Betracht. Bevorzugtes Anodenmaterial ist Graphit. Als Kathodenmaterialien lassen sich beispielsweise Edelmetalle, wie Platin, Metalle wie Ni, Fe oder Stahl, oder Graphit einsetzen. Die Stromdichten betragen z.B. 0,5 - 25 A/dm², bevorzugt wird bei 2 - 10 A/dm² gearbeitet. Die Temperaturen während der Elektrolyse lassen sich in weiten Grenzen wählen, bevorzugt elektrolysiert man mindestens 5°C unter dem Siedepunkt der niedrigsiedendsten Komponente des Elektrolyten. Ein wesentlicher Vorteil des Verfahrens besteht darin, daß die zur Elektrooxidation eingesetzten Verbindungen der Formel II zu über 80 % umgesetzt werden können, ohne daß die Selektivität der Elektrooxidation absinkt. Die Elektrolyte werden nach Beendigung der Elektrolyse durch konventionelle Methoden, wie Destillation, Extraktion und Kristallisation abgetrennt und zur Elektrolyse rückgeführt. Die Elektrosynthesen können sowohl diskontinuierlich als auch kontinuierlich durchgeführt werden.

Die neuen Tetralinderivate der Formel III lassen sich z.B. für die Herstellung der Aldehyde der Formel

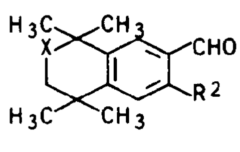

VI,

in der X und $R^2$ die oben genannten Bedeutung haben, verwenden.

Man erhält die neuen Aldehyde durch eine an sich bekannte Hydrolyse, indem man die Acetale der Formel III z.B. in Wasser auf Temperaturen von 30 bis 100°C erhitzt. Dabei kann man vorteilhafterweise auch die rohen Acetale, wie man sie aus der Elektrolyse erhält, ohne Zwischenreinigung zu den neuen Aldehyden hydrolysieren.

Beispiel 1

Elektrosynthese von 7-Acetyl-1,2,3,4-tetrahydro-2-hydroxi-1,1,4,4-tetramethylnaphthalin

| | | |
|---|---|---|
| Apparatur: | | ungeteilte Zelle mit 11 Elektroden |
| Anode: | | Graphit |
| Elektrolyt: | 297 g | 7-Ethyl-1,2,3,4-tetrahydro-2-hydroxi-1,1,4,4-tetramethylnaphthalin |
| | 6 g | $C_6H_5SO_3H$ |
| | 15 g | $NaSO_3C_6H_5$ |
| | 300 g | $H_2O$ |
| | 2379 g | $CH_3OH$ |
| Kathode: | | Graphit |
| Elektrolyse mit 9F/Mol | | 7-Ethyl-1,2,3,4-tetrahydro-2-hydroxi-1,1,4,4-tetramethylnaphthalin |
| Stromdichte: | | 2 A/dm$^2$ |
| Elektrolysetemperatur: | | 40 °C |

Der Elektrolyt wird während der Elektrolyse mit 400 l/h durch die Zelle über einen Wärmetauscher gepumpt

Aufarbeitung:

Nach Beendigung der Elektrolyse werden Methanol und Wasser bei Normaldruck und Sumpftemperaturen bis 120°C abdestilliert. Man extrahiert den Rückstand mit Methyl-tert.-butylether, trennt die Methyl-tert.-butyletherphase ab und destilliert den Methyl-tert.-butylether ab. Der erhaltene Rückstand (322 g) enthält nach der GC-Analyse

2,3 g der Ausgangsverbindung,
22,8 g 7-(2-Methoxy)ethyl-1,2,3,4-tetrahydro-2-hydroxi-1,1,4,4-tetramethylnaphthalin sowie
264 g 7-acethyl-1,2,3,4-tetrahydro-2-hydroxi-1,1,4,4-tetramethylnaphthalin.

Hieraus errechnet sich ein Umsatz von 99,2 %, eine Ausbeute von 83,8 % sowie eine Selektivität von 90,7 %. Der Rückstand kann aus Petrolether/Essigester (5 : 1) umkristallisiert werden. Das 7-Acetyl-1,2,3,4-tetrahydro-2-hydroxi-1,1,4,4-tetramethylnaphthalin (Fp. 98 bis 99°C) wurde durch
$^1$H-NMR (250 MHz, $CDCl_3$): $\delta$ = 1,22(s,3H), 1,35(s,3H), 1,40(s,3H), 1,48(s,3H), 1,75(sbr.,1H), 1,80(dd, $J_1$=12Hz,$J_2$=4Hz,1H), 1,95(t,J=12Hz,1H), 2,62(s,3H), 3,92(dd,$J_1$=12Hz,$J_2$=4Hz,1H), 7,43(d,J=8HZ,1H), 7,80(d,J=8Hz,1H), 8,03(s,1H).
sowie $^{13}$C-NMR
charakterisiert.

Auf analoge Weise wurde 7-Acetyl-1,2,3,4-tetrahydro-1,1,4,4-tetramethyl-2-oxo-naphthalin aus 7-Ethyl-1,2,3,4-tetrahydro-1,1,4,4-tetramethyl-2-oxo-naphthalin hergestellt (Fp. 121 -124°C)
$^1$H-NMR (250 MHz,$CDCl_3$): $\delta$ = 1,34(s,6H), 1,49(s,6H), 2,62(s,3H), 2,66(s, 2H), 7,49(d,J=8Hz,1H), 7,81(d,J=8HZ,1H), 7,98(s,1H).

Beispiel 2

Elektrosynthese von 1,2,3,4-Tetrahydro-2-hydroxi-7-dimethoxymethyl-1,1,4,4-tetramethylnaphthalin

| | |
|---|---|
| Apparatur: | ungeteilte Zelle mit 11 Elektroden |
| Anode: | Graphit |

| | | |
|---|---|---|
| Elektrolyt: | 378 g | 1,2,3,4-Tetrahydro-2-hydroxi-1,1,4,4,7-pentamethylnaphthalin |
| | 15 g | $NaSO_3C_6H_5$ |
| | 2607 g | $CH_3OH$ |

| | |
|---|---|
| Kathode: | Graphit |
| Elektrolyse mit 6F/Mol | 1,2,3,4-Tetrahydro-2-hydroxi-1,1,4,4,7-pentamethylnaphthalin |
| Stromdichte: | 3,3 $A/dm^2$ |
| Elektrolysetemperatur: | 20 bis 25°C |

Der Elektrolyt wird während der Elektrolyse mit 200 l/h durch die Zelle über einen Wärmetauscher gepumpt.

Aufarbeitung:

Nach Beendigung der Elektrolyse werden Methanol bei Normaldruck und Sumpftemperaturen bis 120°C abdestilliert und der Rückstand bei 40°C zur Abtrennung des Leitsalzes filtriert. Danach erhält man 518 g eines Rohacetals, das nach Destillation einer Probemenge zu 76 Gew.% aus 1,2,3,4-Tetrahydro-2-hydroxi-7-dimethoximethyl-1,1,4,4-tetramethylnaphthalin (Kp: 156 bis 160°C bei 2 mbar) besteht. Das erhaltene Produkt wurde durch
$^1$H-NMR (250 MHz, $CDCl_3$): $\delta$ = 1,18(s,3H), 1,30(s,3H), 1,35(s,3H), 1,43(s,3H), 1,75(dd, $J_1$=12Hz, $J_2$=4Hz,1H), 1,87(sbr.,1H), 1,92(t,J=12Hz,1H), 3,37(s,6H), 3,92(d,J=12Hz,1H), 5,38(s,1H), 7,27(d,J=4Hz,1H), 7,33(d,J=4Hz,1H), 7,45(s,1H)
charakterisiert. Es wird eine Ausbeute von 81,7% errechnet.

Beispiel 3:

Synthese von 7-Formyl-1,2,3,4-tetrahydro-2-hydroxi-1,1,4,4-tetramethylnaphthalin

Man legt 360 g Wasser und 0,5 g konzentrierte Schwefelsäure vor und gibt 120 g 1,2,3,4-Tetrahydro-2-hydroxi-7-dimethoximethyl-1,1,4,4-tetramethylnaphthalin zu. Dann erhitzt man auf Rückflußtemperatur und destilliert das Methanol ab. Die organische Phase wird mit Methyl-tert.-butylether aufgenommen. Man trennt die Methyl-tert.-butyletherphase ab, destilliert den Methyl-tert.-butylether ab und kristrallisiert mit Petrolether-/Toluol aus.

Hierbei erhält man 89 g 7-Formyl-1,2,3,4-tetrahydro-2-hydroxi-1,1,4,4-tetramethylnaphthalin (Fp. 76 bis 78°C). Das erhaltene Produkt wurde durch
$^1$H-NMR (250 MHz, $CDCl_3$): $\delta$ = 1,23(s,3H), 1,37(s,3H), 1,41(s,3H), 1,50(s,1H), 1,83(dd, $J_1$=12Hz, $J_2$=4Hz,1H), 1,97(t,J=12Hz,1H), 2,83(sbr.,1H), 3,95(dd,$J_1$=12Hz, $J_2$=4Hz,1H), 7,52(d,J=8Hz, 1H), 7,73(d,J=8HZ,1H), 7,97(s,1H), 10,03(s,1H)
charakterisiert. Die Ausbeute beträgt 88,9%.

Beispiel 4:

Elektrosynthese von 1,2,3,4-Tetrahydro-6-dimethoximethyl-1,1,4,4-tetramethylnaphthalin

| | | |
|---|---|---|
| Apparatur: | | ungeteilte Zelle mit 11 Elektroden |
| Anode: | | Graphit |
| Elektrolyt: | 1200 g | 1,2,3,4-Tetrahydro-1,1,4,4,6-pentamethylnaphthalin |
| | 40 g | $C_6H_5SO_3Na$ |
| | 6760 g | $CH_3OH$ |
| Kathode: | | Graphit |
| Elektrolyse mit 5F/Mol | | 1,2,3,4-Tetrahydro-1,1,4,4,6-pentamethylnaphthalin |
| Stromdichte: | | 3,3 $A/dm^2$ |
| Elektrolysetemperatur: | | 25 °C |

Der Elektrolyt wird während der Elektrolyse mit 400 l/h durch die Zelle über einem Wärmetauscher gepumpt.

Aufarbeitung:

Nach Beendigung der Elektrolyse wird Methanol bei Normaldruck und Sumpftemperaturen bis 120°C abdestilliert. Der Rückstand wird zur Abtrennung von $C_6H_5SO_3Na$ (kann zur Elektrolyse rückgeführt werden) filtriert. Das Filtrat wird bei 2 mbar (Kopfdruck) und 105 bis 125°C (Kopftemperaturen) fraktioniert destilliert. Der Hauptlauf aus 1,2,3,4-tetrahydro-6-dimethoximethyl-1,1,4,4-tetramethylnaphthalin geht bei 112°C über. Nach Destillation erhält man

| | |
|---|---|
| 8,8 g | 1,2,3,4-Tetrahydro-1,1,4,4,6-pentamethylnaphthalin, |
| 81.8 g | 1,2,3,4-Tetrahydro-6-methoximethyl-1,1,4,4-tetramethylnaphtalin, die zur Elektrolyse rückgeführt werden können, |
| 1137,3 g | 1,2,3,4-Tetrahydro-6-dimethoximethyl-1,1,4,4-tetramethylnaphthalin. |

Hieraus errechnen sich:

| | | |
|---|---|---|
| Umsatz: | 99,3 % | 1,2,3,4-Tetrahydro-1,1,4,4,6-pentamethylnaphthalin |
| Ausbeute: | 5,9 % | 1,2,3,4-Tetrahydro-6-methoximethyl-1,1,4,4-tetramethylnaphthalin |
| Ausbeute: | 73,1 % | 1,2,3,4-Tetrahydro-6-dimethoximethyl-1,1,4,4-tetramethylnaphthalin |
| Selektivität. | 78,3 % | 1,2,3,4-Tetrahydro-6-dimethoximethyl-1,1,4,4-tetramethylnaphthalin |

Beispiel 5:

Elektrosynthese von 1,2,3,4-Tetrahydro-6-dimethoximethyl-1,1,4,4,7-pentamethylnaphthalin

| | | |
|---|---|---|
| Apparatur: | | ungeteilte Zelle mit 6 Elektroden |
| Anode: | | Graphit |
| Elektrolyt: | 108 g | 1,2,3,4-Tetrahydro-1,1,4,4,6,7-hexamethyl-naphthalin |
| | 6,5 g | $H_2SO_4$ |
| | 3480 g | $CH_3OH$ |
| Kathode: | | Graphit |
| Elektrolyse mit 3,5 F/Mol | | 1,2,3,4-Tetrahydro-1,1,4,4,6,7-hexamethylnaphthalin |
| Stromdichte: | | 3,3 $A/dm^2$ |
| Elektrolysetemperatur: | | 25°C |

Der Elektrolyt wird während der Elektrolyse mit 200 l/h durch die Zelle über einen Wärmetauscher gepumpt.

Aufarbeitung:

Nach Beendigung der Elektrolyse wird der Elektrolyseaustrag mit $NaOCH_3$ neutralisiert. Dann wird Methanol bei Normaldruck und Sumpftemperaturen bis 120°C abdestilliert, der Rückstand abfiltriert und das Filtrat bei 1 mbar Kopfdruck und 105 bis 125°C Kopftemperaturen fraktioniert destilliert. Hierbei erhält man

16,6 g 1,2,3,4-Tetrahydro-1,1,4,4,6,7-hexamethylnaphthalin

24,0 g 1,2,3,4-Tetrahydro-6-methoximethyl-1,1,4,4,7-pentamethylnaphthalin, die zur Elektrolyse rückgeführt werden können und

50,3 g 1,2,3,4-Tetrahydro-6-dimethoximethyl-1,1,4,4,7-pentamethylnaphthalin.

Hieraus errechnen sich:

| | | |
|---|---|---|
| Umsatz: | 84,6 % | 1,2,3,4-Tetrahydro-1,1,4,4,6,7-hexamethylnaphthalin |
| Ausbeute: | 19,5 % | 1,2,3,4-Tetrahydro-6-methoximethyl-1,1,4,4,7-pentamethylnapthalin |
| Ausbeute: | 36,5 % | 1,2,3,4-Tetrahydro-6-dimethoximethyl-1,1,4,4,7-pentamethylnaphthalin |
| Selektivität: | 56,0 % | 1,2,3,4-Tetrahydro-6-dimethoximethyl-1,1,4,4,7-pentamethylnaphthalin |

Beispiel 6

1,2,3,4-Tetrahydro-7-dimethoximethyl-1,1,4,4-tetramethyl-2-oxo-naphthalin

Analog Beispiel 2 wurde 1,2,3,4-Tetrahydro-1,1,4,4,7-pentamethyl-2-oxo-naphthalin zu 1,2,3,4-Tetrahydro-7-dimethoximethyl-1,1,4,4-tetramethyl-2-oxo-naphthalin elektrochemisch oxidiert. (Kp 0,3mbar 150-154°C). Nach Kristallisation aus n-Heptan wurde 1,2,3,4-Tetrahydro-7-dimethoximethyl-1,1,4,4-tetramethyl-2-oxo-naphthalin als weißer Feststoff in 26,7%iger Ausbeute erhalten (Fp. 91-92°C, GC 98%). Das Acetal wurde durch $^{1}H$- und $^{13}C$-NMR charakterisiert.

$^{1}H$-NMR (250 MHz, $CDCl_3$): $\delta$ = 1,32(s,6H), 1,47(s,6H), 2,67(s,2H), 3,38(s,6H), 5,42(s,1H), 7,33(d,J=8Hz,1H), 7,40(s,1H), 7,45(s,1H),

Beispiel 7

7-Formyl-1,2,3,4-tetrahydro-1,1,4,4-tetramethyl-2-oxo-naphthalin

Die Hydrolyse von 1,2,3,4-Tetrahydro-7-dimethoximethyl-1,1,4,4-tetramethyl-2-oxo-naphthalin zu 7-Formyl-1,2,3,4-tetrahydro-1,1,4,4-tetramethyl-2-oxo-naphthalin wurde analog Beispiel 3 durchgeführt. Der Aldehyd wurde als weißer kristalliner Farbstoff in 95%iger Ausbeute erhalten (Fp. 82-84°C, GC 98%), er wurde durch $^1$H- und $^{13}$C-NMR charakterisiert.
$^1$H-NMR (250 MHz, $CDCl_3$): $\delta$ = 1,37(s,6H), 1,52(s,6H), 2,70(s,2H), 7,63(d,J=8Hz,1H), 7,80(d,J=8Hz,1H), 7,93(s,1H), 10,08(s,1H),

**Patentansprüche**

1. Verfahren zur Herstellung von Tetralinderivaten der Formel

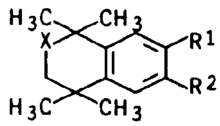

I,

in der
X eine $-CH_2-$, -CH(OH)- oder -CO-Gruppe,
$R^1$ eine der Gruppen $-COCH_3$, $-CH(OR^3)_2$ oder -CHO,
$R^2$ ein Wasserstoffatom oder die Methylgruppe und
$R^3$ einen Alkylrest mit 1 bis 4 C-Atomen
bedeuten, dadurch gekennzeichnet, daß man Verbindungen der Formel

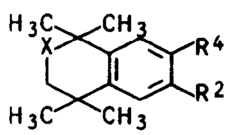

II,

ind der
$R^4$ eine Methyl- oder Ethylgruppe bedeutet und
X und $R^2$ die oben genannte Bedeutung haben, elektrochemisch oxidiert.

2. Verfahren zur Herstellung von Tetralinderivaten der Formel I, in der $R^1$ eine Acetylgruppe bedeutet nach Anspruch 1, dadurch gekennzeichnet, daß man eine Ausgangsverbindung der Formel II, in der $R^4$ eine Ethylgruppe bedeutet und X und $R^2$ die in Anspruch 1 genannte Bedeutung haben, in wäßrigem Medium elektrochemisch oxidiert.

3. Verfahren zur Herstellung von Tetralinderivaten der Formel I, in der $R^1$ den Rest der Formel $-CH(OR^3)_2$ bedeutet, dadurch gekennzeichnet, daß man eine Ausgangsverbindung der Formel II, in der $R^4$ für eine Methylgruppe steht und X und $R^2$ die in Anspruch 1 genannte Bedeutung haben, in Gegenwart eines Alkanols der Formel $R^3OH$, in der R für einen Alkylrest mit 1 bis 4 C-Atomen bedeutet, elektrochemisch oxidiert.

4. Tetralinderivate der Formel

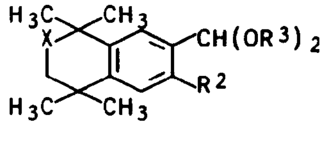

III,

in der
X eine $-CH_2-$, -CH(OH)- oder -CO-Gruppe,
$R^2$ ein Wasserstoffatom oder die Methylgruppe und
$R^3$ eine Alkylgruppe mit 1 bis 4 C-Atomen

bedeuten.

5. Tetralinderivate der Formel

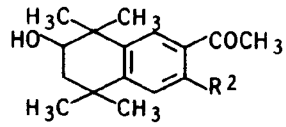

IV,

in der
$R^2$ die in Anspruch 4 genannte Bedeutung hat.

6. Tetralinderivate der Formel

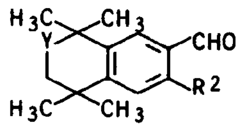

V,

in der
Y eine -CH(OH)- oder -CO-Gruppe bedeutet, und
$R^2$ die in Anspruch 4 genannte Bedeutung
hat.

7. 7-Acetyl-1,2,3,4-tetrahydro-2-hydroxi-1,1,4,4-tetramethylnaphthalin.

8. 7-Formyl-1,2,3,4-tetrahydro-1,1,4,4-tetramethyl-2-oxo-naphthalin.

9. 7-Formyl-1,2,3,4-tetrahydro-2-hydroxi-1,1,4,4-tetramethylnaphthalin.

10. Verwendung von Tetralinderivaten nach Anspruch 4 für die Herstellung von Verbindungen der Formel

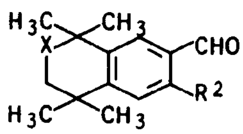

VI,

in der
X und $R^2$ die in Anspruch 4 genannte Bedeutung haben.

## Claims

1. A process for the preparation of a tetralin derivative of the formula

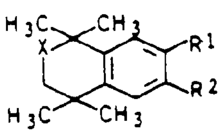

I

where
X is a $-CH_2-$, -CH(OH)- or -CO- group,
$R^1$ is one of the groups $-COCH_3$, $-CH(OR^3)_2$ or -CHO,
$R^2$ is hydrogen or methyl and
$R^3$ is alkyl of 1 to 4 carbon atoms, wherein a compound of the formula

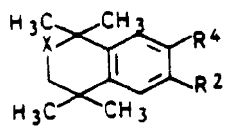

II

where
$R^4$ is methyl or ethyl and
X and $R^2$ have the abovementioned meanings, is subjected to electrochemical oxidation.

2. A process for the preparation of a tetralin derivative of the formula I, where $R^1$ is acetyl, as claimed in claim 1, wherein a starting compound of the formula II, where $R^4$ is ethyl and X and $R^2$ have the meanings stated in claim 1, is subjected to electrochemical oxidation in an aqueous medium.

3. A process for the preparation of a tetralin derivative of the formula I, where $R^1$ is a radical of the formula $-CH(OR^3)_2$, wherein a starting compound of the formula II, where $R^4$ is methyl and X and $R^2$ have the meanings stated in claim 1, is subjected to electrochemical oxidation in the presence of an alkanol of the formula $R^3OH$, where R is alkyl of 1 to 4 carbon atoms.

4. A tetralin derivative of the formula

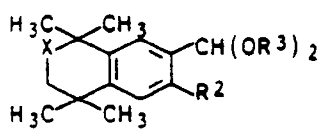

III

where
X is a $-CH_2-$, -CH(OH)- or -CO- group,
$R^2$ is hydrogen or methyl and
$R^3$ is alkyl of 1 to 4 carbon atoms.

5. A tetralin derivative of the formula

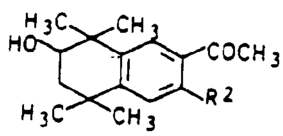

IV

where $R^2$ has the meanings stated in claim 4.

6. A tetralin derivative of the formula

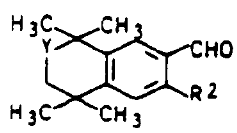

V

where
Y is a -CH(OH)- or -CO- group and
$R^2$ has the meanings stated in claim 4.

7. 7-Acetyl-1,2,3,4-tetrahydro-2-hydroxy-1,1,4,4-tetramethylnaphthalene.

8. 7-Formyl-1,2,3,4-tetrahydro-1,1,4,4-tetramethyl-2-oxonaphthalene.

9. 7-Formyl-1,2,3,4-tetrahydro-2-hydroxy-1,1,4,4-tetramethylnaphthalene.

10. Use of a tetralin derivative as claimed in claim 4 for the preparation of a compound of the formula

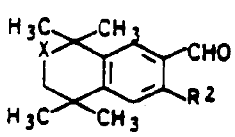

VI

where
X and $R^2$ have the meanings stated in claim 4.

## Revendications

1. Procédé de préparation de dérivés de tétraline de formule

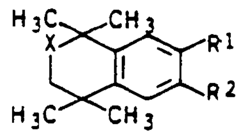

I,

dans laquelle
X représente un groupement $-CH_2-$, -CH(OH)- ou -CO-,
$R^1$ réprésente l'un des groupements $-COCH_3$, $-CH(OR^3)_2$ ou -CHO,
$R^2$ représente un atome d'hydrogène ou un groupement méthyle et
$R^3$ représente un reste alkyle ayant de 1 à 4 atomes de carbone,
caractérisé en ce qu'on oxyde par voie électrochimique des composés de formule

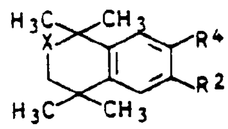

II,

dans laquelle
$R^4$ représente un groupement méthyle ou éthyle et
X et $R^2$ ont les significations données ci-dessus.

2. Procédé de préparation de dérivés de tétraline de formule I dans laquelle $R^1$ est un groupement acétyle, selon la revendication 1, caractérisé en ce qu'on oxyde par voie électrochimique un composé de départ de formule II dans laquelle $R^4$ représente un groupement éthyle et X et $R^2$ ont les significations données dans la revendication 1.

3. Procédé de préparation de dérivés de tétraline de formule I dans laquelle $R^1$ représente le reste de formule $-CH(OR^3)_2$, caractérisé en ce que l'on oxyde par voie électrochimique un composé de départ de formule II dans laquelle $R^4$ est mis pour un groupement méthyle et X et $R^2$ ont les significations données dans la revendication 1, en présence d'un alcool de formule $R^3OH$ dans laquelle R représente un groupement alkyle ayant de 1 à 4 atomes de carbone.

4. Dérivés de tétraline de formule

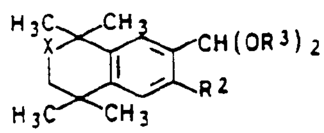

III,

dans laquelle
X représente un groupement $-CH_2-$, -CH(OH)- ou -CO-,
$R^2$ représente un atome d'hydrogène ou un groupement méthyle et
$R^3$ représente un groupement alkyle ayant de 1 à 4 atomes de carbone.

5. Dérivés de tétraline de formule

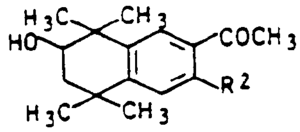

IV,

dans laquelle
$R^2$ a la signification donnée dans la revendication 4.

6. Dérivés de tétraline de formule

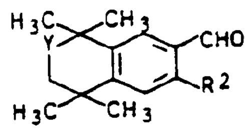

V,

dans laquelle
Y représente un groupement -CH(OH)- ou -CO-, et
$R^2$ a la signification donnée dans la revendication 4.

7. 7-Acétyl-1,2,3,4-tétrahydro-2-hydroxy-1,1,4,4-tétraméthylnaphtalène.

8. 7-Formyl-1,2,3,4-tétrahydro-1,1,4,4-tétraméthyl-2-oxo-naphtalène.

9. 7-Formyl-1,2,3,4-tétrahydro-2-hydroxy-1,1,4,4-tétraméthylnaphtalène.

10. Utilisation des dérivés de tétraline selon la revendication 4 pour la préparation de dérivés de formule

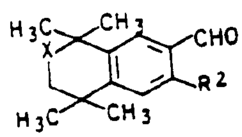

VI,

dans laquelle
X et $R^2$ ont les significations données dans la revendication 4.